Europäisches Patentamt

European Patent Office

Office européen des brevets

(19)

(11) Publication number: **0 153 781**
**B1**

(12) **EUROPEAN PATENT SPECIFICATION**

(45) Date of publication of patent specification: **05.10.88**

(21) Application number: **85200183.3**

(22) Date of filing: **12.02.85**

(51) Int. Cl.⁴: **C 07 C 1/04,** B 01 J 23/80, C 07 C 1/06

(54) Process for the preparation of hydrocarbons.

(30) Priority: **28.02.84 NL 8400608**

(43) Date of publication of application:
**04.09.85 Bulletin 85/36**

(45) Publication of the grant of the patent:
**05.10.88 Bulletin 88/40**

(84) Designated Contracting States:
**AT BE DE FR IT NL SE**

(56) References cited:
**EP-A-0 127 220**
**GB-A-2 077 289**
**GB-A-2 125 062**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Post, Martin Franciscus Maria**
**Badhuisweg 3**
**NL-1031 CM Amsterdam (NL)**
Inventor: **Sie, Swan Tiong**
**Badhuisweg 3**
**NL-1031 CM Amsterdam (NL)**

(74) Representative: **Aalbers, Onno et al**
**P.O. Box 302**
**NL-2501 CH The Hague (NL)**

Courier Press, Leamington Spa, England.

## Description

The invention relates to a process for the preparation of hydrocarbons by catalytic conversion of a mixture of carbon monoxide and hydrogen.

The preparation of hydrocarbons from a $H_2/CO$ mixture by contacting this mixture at elevated temperature and pressure with a catalyst is known in the literature as the Fischer-Tropsch hydrocarbon synthesis. Catalysts often used for the purpose comprise one or more metals from the iron group, together with one or more promoters, and a carrier material. These catalysts can suitably be prepared by the known techniques, such as precipitation, impregnation, kneading and melting. The products which can be prepared by using these catalysts generally have a very wide molecular weight distribution range and, in addition to branched and unbranched paraffins, they often contain considerable amounts of olefins and oxygen-containing organic compounds. Usually only a minor portion of the products obtained is made up of middle distillates. Of these middle distillates not only the yield but also the pour point is unsatisfactory. Therefore the direct conversion of $H_2/CO$ mixtures according to Fischer-Tropsch is not a very attractive route for the production of middle distillates on a technical scale.

In this patent application "middle distillate" should be taken to be hydrocarbon mixtures whose boiling range corresponds substantially with that of the kerosine and gas oil fractions obtained in the conventional atmospheric distillation of crude mineral oil. The middle distillate range lies substantially between about 150 and 360°C.

Recently there was found a class of Fischer-Tropsch catalysts having the property of yielding a product in which only very minor amounts of olefins and oxygen-containing compounds occur and which consists virtually completely of unbranched paraffins, a considerable portion of which paraffins boils above the middle distillate range. It has been found that by hydrocracking the high-boiling part of this product can be converted in high yield into middle distillates. As feed for the hydrocracking at least the part of the product is chosen whose initial boiling point lies above the final boiling point of the heaviest middle distillate desired as end product. The hydrocracking, which is characterized by a very low hydrogen consumption, leads to middle distillates which have a considerably better pour point than those obtained in the direct conversion of a $H_2/CO$ mixture according to Fischer-Tropsch.

The Fischer-Tropsch catalysts belonging to the afore-mentioned class contain silica, alumina or silica-alumina as carrier material and cobalt together with zirconium, titanium and/or chromium as catalytically active metals, in such quantities that per 100 pbw of carrier material the catalysts comprise 3—60 pbw of cobalt and 0.1—100 pbw of zirconium, titanium or chromium. The catalysts are prepared by depositing the metals involved on the carrier material by kneading and/or impregnation. For further information on the preparation of these catalysts by kneading and/or impregnation reference may be made to Netherlands Patent Application No. 8301922 recently filed in the name of the Applicant.

When the present cobalt catalysts are used for the Fischer-Tropsch hydrocarbon synthesis starting from $H_2/CO$-mixtures having a $H_2/CO$ molar ratio of about 2, very high $H_2+CO$ conversions can be achieved. However, when feeds with a lower $H_2/CO$ molar ratio are used, the $H_2+CO$ conversion is insufficient. The $H_2+CO$ conversion is seen to be lower according as the feed has a lower $H_2/CO$ molar ratio.

Since nature provides large amounts of material with a relatively low H/C ratio, such as coal, which when converted into $H_2/CO$ mixtures yields products having a $H_2/CO$ molar ratio lower than 2, it would naturally be very welcome if a way could be found to solve the afore-mentioned problem of low $H_2+CO$ conversions.

During an investigation into this subject two measures were found which have made it possible to realise high $H_2+CO$ conversions in the hydrocarbon synthesis starting from $H_2/CO$ mixtures having $H_2/CO$ molar ratios between 0.25 and 1.75 and by using the present cobalt catalysts. In addition the application of these measures leads to a high $C_5^+$ selectivity. By the first measure the $H_2/CO$ mixture is converted over a mixture of two catalysts, one of which is the cobalt catalyst and the other a copper- and zinc-containing composition. By the second measure the $H_2/CO$ mixture is first partly converted over the cobalt catalyst, and subsequently the unconverted $H_2$ and CO is converted over a bifunctional catalyst or catalyst combination which, in addition to activity for the conversion of a $H_2/CO$ mixture into hydrocarbons, has activity for the conversion of a mixture of $H_2O$ and CO into a mixture of $H_2$ and $CO_2$.

Conditional upon the $H_2/CO$ molar ratio of the feed to be converted it is either exclusively measure 1, or exclusively measure 2, or either one of the two measures that is eligible for use. For feeds with a $H_2CO$ molar ratio between 0.25 and 0.75 only measure 1 is applicable. If the feed has a $H_2/CO$ molar ratio between 1.0 and 1.75, it is only measure 2 that is eligible. For feeds with a $H_2/CO$ molar ratio between 0.75 and 1.0 either measure 1 or measure 2 can be used at choice. GB—A—2,077,289 describes a process for the preparation of hydrocarbons from a mixture of CO and $H_2$ by contacting the mixture with the above Fischer-Tropsch catalyst, prepared by impregnation, or with such a catalyst in combination with a CO-shift catalyst.

The present patent application relates to the use of measure 2 for feeds with a $H_2/CO$ molar ratio between 0.75 and 1.75.

The partial conversion which in accordance with measure 2 is carried out in the first step of the process should be performed under such conditions as to satisfy the relation

$$150 \times \frac{F-0.5}{F+1} < C < 250 \times \frac{F-0.5}{F+1},$$

wherein F represents the $H_2$/CO molar ratio of the feed and C the $H_2$+CO conversion, expressed as %mol. After the water formed has been removed from the product of the first step at least the unconverted $H_2$ and CO thereof should be contacted in the second step with the bifunctional catalyst or catalyst combination mentioned hereinbefore.

The present patent application therefore relates to a process for the preparation of hydrocarbons by catalytic reaction of carbon monoxide with hydrogen, in which a $H_2$- and CO-containing feed is contacted in the first step with a catalyst comprising 3—60 pbw of cobalt and 0.1—100 pbw of at least one other metal chosen form the group formed by zirconium, titanium and chromium per 100 pbw of silica, alumina or silica-alumina, which catalyst has been prepared by kneading and/or impregnation, characterized in that the feed has a $H_2$/CO molar ratio (F) in the range between 0.75 and 1.75, and the contact is brought about under such conditions as to satisfy the relation

$$150 \times \frac{F-0.5}{F+1} < C < 250 \times \frac{F-0.5}{F+1},$$

wherein C represents the $H_2$+CO conversion expressed as %mol, and that of the product from the first step—after removal of the water formed—at least the $H_2$ and CO that has remained unconverted is contacted in a second step with a catalyst or catalyst combination which, in addition to activity for the conversion of a mixture of $H_2$ and CO into hydrocarbons, has activity for the conversion of a mixture of $H_2O$ and CO into a mixture of $H_2$ and $CO_2$.

In the process of the invention it is preferred to use in the first step the cobalt catalysts which form the subject matter of Netherlands patent application No. 8301922. These are catalysts which satisfy the relation

$$(3+4R) > \frac{L}{S} > (0.3+0.4R),$$

wherein
   L=the total quantity of cobalt present in the catalyst, expressed as mg Co/ml catalyst,
   S=the surface area of the catalyst, expressed as $m^2$/ml catalyst, and
   R=the weight ratio of the quantity of cobalt deposited on the catalyst by kneading to the total quantity of cobalt present in the catalyst.

The preparation of the cobalt catalysts used in the first step of the process of the invention is preferably carried out by one of the three procedures mentioned hereinafter:
   a) first cobalt is deposited in one or more steps by impregnation and subsequently the other metal is deposited in one or more steps, also by impregnation,
   b) first the other metal is deposited in one or more steps by impregnation and subsequently the cobalt is deposited in one or more steps, also by impregnation, and
   c) first cobalt is deposited in one or more steps by kneading and subsequently the other metal is deposited in one or more steps by impregnation.

In the process according to the invention preference is given to the use of cobalt catalysts containing 15—50 pbw of cobalt per 100 pbw of carrier. The preferred quantity of other metal present in the cobalt catalysts depends on the way in which this metal has been deposited. In the case of catalysts where first cobalt has been deposited on the carrier, followed by the other metal, preference is given to catalysts containing 0.1—5 pbw of the other metal per 100 pbw of carrier. In the case of catalysts where first the other metal has been deposited on the carrier, followed by the cobalt, preference is given to catalysts containing 5—40 pbw of the other metal per 100 pbw of carrier. Preference is given to zirconium as other metal and to silica as carrier material. Preparatory to being suitable for use the cobalt catalysts should first be activated. This activation may suitably be carried out by contacting the catalyst at a temperature between 200 and 350°C with hydrogen or a hydrogen-containing gas.

In the process according to the invention from the product of the first step—after removal of the water formed—at least the $H_2$ and CO that has remained unconverted is contacted in a second step with the bifunctional catalyst or catalyst combination mentioned hereinbefore. It is preferred to divide the product of the first step by cooling into a gaseous fraction substantially consisting of unconverted $H_2$ and CO, and $C_4^-$ hydrocarbons, and a liquid fraction substantially consisting of $C_5^+$ hydrocarbons and water, and to use the gaseous fraction as feed for the second step. Optionally the total reaction product of the first step may be used—after removal of water—as feed for the second step.

As examples of catalysts and catalyst combinations eligible for use in the second step of the process according to the invention may be mentioned:—
   1) Catalysts prepared by impregnation which comprise 5—50%w of iron and 5—50%w of copper and zinc supported on a carrier and in which the weight ratio of the total quantity of copper and zinc to the quantity of iron is 0.5—5. When such catalysts are used preference is given to catalysts whose Cu/Zn atomic ratio lies between 0.25 and 4 and in which the weight ratio of the total quantity of copper and zinc to the quantity of iron lies between 1.0 and 3.0. Examples of such catalysts are $Fe/K/Cu/Zn/SiO_2$ catalysts.

2) Catalysts prepared by impregnation which comprise 30—75 pbw of iron and 5—40 pbw of magnesium per 100 pbw of alumina. When such catalysts are used preference is given to catalysts comprising 40—60 pbw of iron and 7.5—30 pbw of magnesium per 100 pbw of alumina. Examples of such catalysts are $Fe/K/Cu/Mg/Al_2O_3$ catalysts.

3) Catalysts prepared by impregnation which comprise 10—40 pbw of iron and 0.25—10 pbw of chromium per 100 pbw of silica. When such catalysts are used preference is given to catalysts comprising 20—35 pbw of iron and 0,5—5 pbw of chromium per 100 pbw of silica. Examples of such catalysts are $Fe/K/Cr/SiO_2$.

4) Catalyst mixtures obtained by mixing a crystalline metal silicate of a special structure either with one of the iron catalysts mentioned under 1)—3), or with a $ZnO/Cr_2O_3$ catalyst, or with a $Cu/ZnO/Cr_2O_3$ catalyst, or with a mixture of a $Cu/ZnO/Cr_2O_3$ catalyst and gamma-$Al_2O_3$.

The crystalline metal silicates mentioned under 4) are characterized in that after one hour's calcination in air at 500°C, they have the following properties

a) an X-ray powder diffraction pattern in which the four lines mentioned in Table A are the strongest lines

TABLE A
d(Å)

11.1 ±0.2
10.0 ±0.2
3.84±0.07
3.72±0.06,

and

b) in the formula which represents the composition of the silicate expressed in moles of the oxides and in which, in addition to $SiO_2$, one or more oxides of a trivalent metal M, chosen from the group formed by aluminium, iron and gallium, are present, the $SiO_2/M_2O_3$ molar ratio is higher than 10.

Since it is desirable to prepare in the process according to the invention so large an amount as possible of a product which can be converted into middle distillates by hydrocracking, it is preferred to use in the second step a catalyst combination in which the activity for the conversion of a $H_2/CO$ mixture into hydrocarbons originates in a cobalt catalyst belonging to the same class as that from which the cobalt catalyst used in the first step was chosen. When such a cobalt catalyst is used in the second step, the same preference applies with regard to method of preparation and composition as mentioned for the cobalt catalyst used in the first step.

In the process according to the invention preference is given in the second step to the use of a mixture of two catalysts, the one catalyst being a cobalt catalyst belonging to the same class as that from which the cobalt catalyst used in the first step was chosen, and the other catalyst being a copper- and zinc-containing composition having a Cu/Zn atomic ratio between 0.1 and 10. Special preference is given to such catalyst mixtures wherein the two catalysts are present in such a ratio that the (Cu+Zn)/Co atomic ratio in the catalyst mixture lies between 0.5 and 5. The copper- and zinc-containing composition present in the catalyst mixture preferably has a Cu/Zn atomic ratio between 0.25 and 4. Preparatory to being eligible for use the catalyst mixtures containing a cobalt catalyst and a copper- and zinc-containing composition should be activated. This activation may suitably be carried out by contacting the catalyst mixture with hydrogen or a hydrogen-containing gas, first at a temperature between 150 and 250°C and subsequently at a higher temperature, between 200 and 350°C.

The process according to the invention is preferably carried out at a temperature of 125—350°C and a pressure of 5—100 bar. Special preference is given to a temperature of 175—275°C and a pressure of 10—75 bar.

As stated hereinbefore, the present cobalt catalysts when used for the conversion of a $H_2$- and CO-containing feed yield a substantially waxy product whose high-boiling part can be converted in high yield into middle distillates by a hydrocracking treatment. This is also true when use is made not of the cobalt catalysts alone but of the above-described catalyst mixtures containing such cobalt catalysts.

Although, when the products prepared over the present cobalt catalysts or over catalyst mixtures containing the present cobalt catalysts are used in the preparation of middle distillates, the part of those products whose initial boiling point lies above the final boiling point of the heaviest middle distillate desired as end product will do as feed for the hydrocracking, it is preferred to use for this purpose the total $C_5^-$ fraction of said products, since it has been found that the catalytic hydrotreatment leads to enhanced quality of the gasoline, kerosine and gas oil fractions present therein.

The hydrocracking is carried out by contacting the fraction to be treated at elevated temperature and pressure and in the presence of hydrogen with a catalyst comprising one or more noble metals from Group VIII supported on a carrier. The hydrocracking catalyst used by preference is a catalyst comprising 0.1—2%w, and in particular 0.2—1%w, of one or more noble metals from Group VIII supported on a carrier.

4

Preference is given to catalysts containing platinum or palladium as Group VIII noble metal and silica-alumina as carrier. The hydrocracking is preferably carried out at a temperature of 200—400°C and in particular of 250—350°C and a pressure of 5—100 bar and in particular of 10—75 bar.

The invention is now illustrated with the aid of the following example.

## Example

Catalyst 1 and Catalyst 2 were mixed together to compose Catalyst Mixture I.

## Catalyst 1

$Co/Zr/SiO_2$ catalyst which comprised 25 pbw of cobalt and 0.9 pbw of zirconium per 100 pbw of silica and had been prepared by single-step impregnation of a silica carrier with a solution of cobalt nitrate in water, followed by single-step impregnation of the cobalt-loaded carrier with a solution of zirconium nitrate in water. In both impregnation steps the quantity of solution used had a volume which corresponded substantially with the pore volume of the carrier. After the two impregnation steps the material was dried and then calcined at 500°C. The catalyst had a value for L of 98 mg/ml and for S of 96 $m^2$/ml, and consequently it had a value for L/S of 1.02 $mg/m^2$.

## Catalyst 2

$Cu/Zn/Al_2O_3$ catalyst comprising 24.3%w of copper and 38.0%w of zinc and therefore having a Cu/Zn atomic ratio of 0.66.

## Catalyst Mixture I

Catalysts 1 and 2 were mixed in such a ratio as to obtain a Catalyst Mixture I, whose (Cu+Zn)/Co atomic ratio was 3.75.

## Catalyst testing

Catalyst 1 and Catalyst Mixture I were used in eight experiments (1—8) in the preparation of hydrocarbons from mixtures of carbon monoxide and hydrogen. The experiments were carried out at a space velocity of 10 Nl feed per hour and at a pressure of 20 bar in one or two reactors containing a fixed catalyst bed of Catalyst 1 or Catalyst Mixture I. Experiments 1 and 2 were carried out in a single step by using Catalyst 1. Experiment 8 was also carried out in a single step, but using Catalyst Mixture I. Experiments 3—7 were carried out in two steps by using Catalyst 1 in the first step and Catalyst Mixture I in the second step. In the two-step experiments the reaction product from the first step was cooled to divide it into a gaseous fraction substantially consisting of unconverted $H_2$ and CO, and $C_4^-$ hydrocarbons, and a liquid fraction substantially consisting of $C_5^+$ hydrocarbons and water, and the gaseous fraction was used as feed for the second step. Preparatory to the testing Catalyst 1 and Catalyst Mixture I were activated by contacting them with a hydrogen-containing gas; Catalyst 1 at 250°C; Catalyst Mixture I first at 200°C and subsequently at 250°C. The results of the experiments and the $H_2$/CO molar ratios (F) of the feeds used in each of the experiments as well as the reaction conditions are listed in the table.

The parameters C, total $H_2$+CO conversion, and total $C_5^+$ selectivity used in the table are defined as follows:—

$$C = \frac{\text{mol } H_2+CO \text{ present in feed} - \text{mol } H_2+CO \text{ present in product of first step}}{\text{mol } H_2+CO \text{ present in feed}} \times 100$$

$$\text{Total } H_2+CO \text{ conversion} = \frac{\text{mol } H_2+CO \text{ present in feed} - \text{mol } H_2+CO \text{ present in product of second step}}{\text{mol } H_2+CO \text{ present in feed}} \times 100$$

$$\text{Total } C_5^+ \text{ selectivity} = \frac{\text{pbw } C_5^+ \text{ hydrocarbons present in product of first+second step}}{\text{pbw hydrocarbons present in product of first+second step}} \times 100$$

## 0 153 781

TABLE

| Exp. No. | Reaction step No. | Quantity of catalyst, ml | F | Temp., °C | C, % mol | Total $H_2+CO$ conversion % mol | Total $C_5^+$ selectivity, %w |
|---|---|---|---|---|---|---|---|
| 1 | — | 20 | 1.0 | 220 | — | 68 | 88 |
| 2 | — | 20 | 1.0 | 230 | — | 74 | 87 |
| 3 | 1 | 6.5 | 1.0 | 230 | 48 | 91 | 86 |
|   | 2 | 13.5 |   | 250 |   |   |   |
| 4 | 1 | 6.5 | 1.0 | 245 | 66 | 81 | 86 |
|   | 2 | 13.5 |   | 250 |   |   |   |
| 5 | 1 | 3 | 1.0 | 220 | 18 | 86 | 75 |
|   | 2 | 17 |   | 250 |   |   |   |
| 6 | 1 | 6.5 | 1.5 | 235 | 76 | 99 | 84 |
|   | 2 | 13.5 |   | 250 |   |   |   |
| 7 | 1 | 3 | 1.5 | 225 | 30 | 77 | 72 |
|   | 2 | 17 |   | 250 |   |   |   |
| 8 | — | 20 | 1.5 | 250 | — | 63 | 55 |

## Claims

1. A process for the preparation of hydrocarbons by catalytic reaction of carbon monoxide with hydrogen, in which a $H_2$- and CO-containing feed is contacted in the first step with a catalyst comprising 3—60 pbw of cobalt and 0.1—100 pbw of at least one other metal chosen from the group formed by zirconium, titanium and chromium per 100 pbw of silica, alumina or silica-alumina, which catalyst has been prepared by kneading and/or impregnation, characterized in that the feed has a $H_2/CO$ molar ratio (F) in the range between 0.75 and 1.75, and the contact is brought about under such conditions as to satisfy the relation

$$150 \times \frac{F-0.5}{F+1} < C < 250 \times \frac{F-0.5}{F+1},$$

wherein C represents the $H_2+CO$ conversion expressed as %mol, and that of the product from the first step—after removal of the water formed—at least the $H_2$ and CO that has remained unconverted is contacted in a second step with a catalyst or catalyst combination which, in addition to activity for the conversion of a mixture of $H_2$ and CO into hydrocarbons, has activity for the conversion of a mixture of $H_2O$ and CO into a mixture of $H_2$ and $CO_2$.

2. A process as claimed in claim 1, characterized in that in the first step a catalyst is used which satisfies the relation

$$(3+4R) > \frac{L}{S} > (0.3+0.4R),$$

wherein

L=the total quantity of cobalt present in the catalyst, expressed as mg Co/ml catalyst,

S=the surface area of the catalyst, expressed as $M^2$/ml catalyst, and

R=the weight ratio of the quantity of cobalt deposited on the carrier by kneading to the total quantity of cobalt present in the catalyst.

3. A process as claimed in claim 1 or 2, characterized in that in the first step use is made of a catalyst which per 100 pbw carrier comprises 15—50 pbw of cobalt and either 0.1—5 pbw of the other metal if during the preparation cobalt was deposited first and the other metal next, or 5—40 pbw of the other metal if in the preparation the other metal was deposited first and cobalt next.

4. A process as claimed in any one of claims 1—6, characterized in that in the first step use is made of a catalyst comprising zirconium as other metal and silica as carrier.

6

5. A process as claimed in any one of claims 1—4, characterized in that the product from the first step is divided by cooling into a gaseous fraction substantially consisting of unconverted $H_2$ and CO and $C_4^-$ hydrocarbons, and a liquid fraction substantially consisting of $C_5^+$ hydrocarbons and water, and that the gaseous fraction is used as feed for the second step.

6. A process as claimed in any one of claims 1—5, characterized in that in the second step use is made of a catalyst combination in which the activity for the conversion of a $H_2$/CO mixture into hydrocarbons orginates in a cobalt catalyst as defined in claim 1.

7. A process as claimed in claim 6, characterized in that in the second step use is made of a mixture of two catalysts, the one catalyst being a cobalt catalyst as defined in claim 1 and the other catalyst being a copper- and zinc-containing composition having a Cu/Zn atomic ratio in the range between 0.1 and 10.

8. A process as claimed in claim 7, characterized in that the two catalysts are present in the catalyst mixture in such a ratio that the (Cu+Zn)/Co atomic ratio in the catalyst mixture lies between 0.5 and 5.

9. A process as claimed in claim 7 or 8, characterized in that the copper- and zinc-containing composition has a Cu/Zn atomic ratio in the range between 0.25 and 4.

10. A process as claimed in any one of claims 1—9, characterized in that it is carried out at a temperature of 125—350°C and a pressure of 5—100 bar.

## Patentansprüche

1. Verfahren zur Herstellung von Kohlenwasserstoffen durch katalytische Umsetzung von Kohlenmonoxid mit Wasserstoff, worin ein $H_2$ und CO enthaltendes Einsatzmaterial in der ersten Stufe mit einem 3 bis 60 Gewichtsteile Kobalt und 0,1 bis 100 Gewichtsteile wenigstens eines anderen Metalles, ausgewählt aus der aus Zirkon, Titan und Chrom umfassenden Gruppe, je 100 Gewichtsteile Siliziumdioxid, Aluminiumoxid oder Siliziumdioxid-Aluminiumoxid enthaltenden Katalysator in Berührung gebracht wird, welcher Katalysator durch Kneten und/oder Imprägnieren hergestellt werden ist, dadurch gekennzeichnet, daß das Einsatzmaterial ein $H_2$/CO-Molverhältnis (F) im Bereich von 0,75 bis 1,75 aufweist und der Kontakt unter solchen Bedingungen herbeigeführt wird, daß die Beziehung

$$150 \times \frac{F-0,5}{F+1} < C < 250 \times \frac{F-0,5}{F+1}$$

erfüllt wird, worin C die $H_2$+CO-Umwandlung, ausgedrückt als Mol-%, bedeutet, und daß von dem Produkt aus der ersten Stufe—nach Abtrennung des gebildeten Wassers—wenigstens der Wasserstoff und das Kohlenmonoxid, die nicht umgewandelt worden sind, in einer zweiten Stufe mit einem Katalysator oder einer Katalysatorkombination in Berührung gebracht werden, der bzw. die zusätzlich zu einer Aktivität für die Umwandlung eines Gemisches aus $H_2$ und CO in Kohlenwasserstoffe eine Aktivität für die Umwandlung eines Gemisches aus $H_2O$ und CO in ein Gemisch aus $H_2$ und $CO_2$ aufweist.

2. Verfahren nach Anspruch 1, dadurch gekennzeichnet, daß in der ersten Stufe ein Katalysator verwendet wird, der die Beziehung

$$(3+4R) > \frac{L}{S} > (0,3+0,4R)$$

erfüllt, worin

L=Gesamtmenge an auf dem Träger vorliegenden Kobalt, ausgedrückt als mg Co/ml Katalysator,
S=Oberfläche des Katalysators, ausgedrückt als $m^2$/ml Katalysator und
R=Gewichtsverhältnis der auf dem Katalysator durch Kneten abgelagerten Kobaltmenge zu der auf dem Katalysator vorliegenden Gesamtmenge an Kobalt.

3. Verfahren nach Anspruch 1 oder 2, dadurch gekennzeichnet, daß in der ersten Stufe ein Katalysator verwendet wird, der je 100 Gewichtsteile Trägermaterial 15 bis 50 Gewichtsteile Kobalt und entweder 0,1 bis 5 Gewichtsteile des anderen Materials enthält, wenn während der Herstellung zuerst das Kobalt und dann das andere Metall abgelagert worden sind, oder 5 bis 40 Gewichtsteile das anderen Metalls enthält, wenn in der Herstellung zuerst das andere Metall und danach das Kobalt abgelagert worden sind.

4. Verfahren nach einem der Ansprüche 1 bis 3, dadurch gekennzeichnet, daß in der ersten Stufe ein Katalysator eingesetzt wird, der Zirkon als das andere Metall und Siliziumdioxid als Trägermaterial aufweist.

5. Verfahren nach einem der Ansprüche 1 bis 4, dadurch gekennzeichnet, daß das Produkt aus der ersten Stufe durch Kühlen in eine gasförmige Fraktion, die im wesentlichen aus nicht umgewandeltem $H_2$ und CO und aus $C_4^-$-Kohlenwasserstoffen besteht, und in eine flüssige Fraktion unterteilt wird, die im wesentlichen aus $C_5^-$-Kohlenwasserstoffen und Wasser besteht, und daß die gasförmige Fraktion als Einsatzmaterial für die zweite Stufe verwendet wird.

6. Verfahren nach einem der Ansprüche 1 bis 5, dadurch gekennzeichnet, daß in der zweiten Stufe eine Katalysatorkombination eingesetzt wird, worin die Aktivität für die Umwandlung eines $H_2$/CO-Gemisches in Kohlenwasserstoffe aus einem Kobaltkatalysator, wie in Anspruch 1 definiert, herrührt.

7. Verfahren nach Anspruch 6, dadurch gekennzeichnet, daß in der zweiten Stufe ein Gemisch aus zwei Katalysatoren eingesetzt wird, wovon der eine Katalysator ein wie in Anspruch 1 definierter Kobaltkatalysator ist und der andere Katalysator eine Kupfer und Zink enthaltende Zusammensetzung mit einem Cu-Zn-Atomverhältnis im Bereich von 0,1 bis 10 ist.

8. Verfahren nach Anspruch 7, dadurch gekennzeichnet, daß die beiden Katalysatoren im Katalysatorgemisch in einem solchen Verhältnis vorliegen, daß das (Cu+Zn)/Co-Atomverhältnis im Katalysatorgemisch im Bereich von 0,5 bis 5 liegt.

9. Verfahren nach Anspruch 7 oder 8, dadurch gekennzeichnet, daß die Kupfer und Zink enthaltende Zusammensetzung ein Cu/Zn-Atomverhältnis im Bereich von 0,25 bis 4 hat.

10. Verfahren nach einem der Ansprüche 1 bis 9, dadurch gekennzeichnet, daß es bei einer Temperatur von 125 bis 350°C und bei einem Druck von 5 bis 100 bar ausgeführt wird.

**Revendications**

1. Un procédé pour la préparation d'hydrocarbures par réaction catalytique de l'oxyde de carbone avec l'hydrogène, dans lequel une charge contenant $H_2$ et CO est mise en contact dans la première étape avec un catalyseur comprenant 3—60 parties en poids de cobalt et 0,1—100 parties en poids d'au moins un autre métal choisi dans le groupe formé par le zirconium, le titane et le chrome pour 100 parties en piods de silice, d'alumine ou de silice-alumine, ce catalyseur ayant été préparé par malaxage et/ou imprégnation, caractérisé en ce que la charge a un rapport molaire $H_2/CO$ (F) compris entre 0,75 et 1,75 et que la mise en contact est effectuée dans des conditions telles que la relation suivante soit satisfaite

$$150 \times \frac{F-0,5}{F+1} < C < 250 \times \frac{F-0,5}{F+1},$$

où C représente la conversion de $H_2+CO$ exprimée en pourcentage molaire, et que, après élimination de l'eau formée, au moins le $H_2$ et le CO non transformés présents dans le produit de la première étape sont mis en contact dans une seconde étape avec un catalyseur ou une combinaison de catalyseurs qui, en plus d'une activité pour la conversion d'un mélange de $H_2$ et CO en hydrocarbures, a une activité pour la conversion d'un mélange de $H_2O$ et CO en un mélange de $H_2$ et $CO_2$.

2. Un procédé selon la revendication 1, caractérisé en ce que dans la première étape on utilise un catalyseur qui satisfait à la relation

$$(3+4R) > \frac{L}{S} > (0,3+0,4R),$$

où

L=la quantité totale de cobalt présente dans le catalyseur, exprimée en mg de Co par ml de catalyseur,
S=l'aire superficielle du catalyseur, exprimée en $m^2$ par ml de catalyseur,
R=le rapport en poids de la quantité de cobalt déposée sur le support par malaxage à la quantité totale de cobalt présente dans le catalyseur.

3. Un procédé selon la revendication 1 ou 2, caractérisé en ce que dans la première étape on utilise un catalyseur qui, pour 100 parties en poids de support, comprend 15—50 parties en poids de cobalt et soit 0,1—5 parties en poids de l'autre métal si durant la préparation le cobalt a été déposé d'abord et l'autre métal ensuite, soit 5—40 parties en poids de l'autre métal si dans la prépartaion l'autre métal a été déposé d'abord et le cobalt ensuite.

4. Un procédé selon l'une quelconque des revendications 1—3, caractérisé en ce que dans la première étape on utilise un catalyseur comprenant du zirconium comme autre mètal et de la silice comme support.

5. Un procédé selon l'une quelconque des revendications 1—4, caractérisé en ce que le produit de la première étape est divisé par refroidissement en une fraction gazeuse constituée substantiellement de $H_2$ et CO non transformés et d'hydrocarbures en $C_4-$, et une fraction liquide constituée substantiellement d'hydrocarbures et $C_5+$ et d'eau, et que la fraction gazeuse est utilisée comme charge pour la seconde étape.

6. Un procédé selon l'une quelconque des revendications 1—5, caractérisé en ce que dans la seconde étape on utilise une combinaison de catalyseurs dans laquelle l'activité pour la conversion d'un mélange $H_2/CO$ en hydrocarbures provient d'un catalyseur au cobalt tel que défini dans la revendication 1.

7. Un procédé selon la revendication 6, caractérisé en ce que dans la seconde étape on utilise un mélange de deux catalyseurs, l'un des catalyseurs étant un catalyseur au cobalt tel que défini dans la revendication 1 et l'autre catalyseur étant une combinaison contenant du cuivre et du zinc ayant un rapport atomique $Cu/Zn$ compris entre 0,1 et 10.

8. Un procédé selon la revendication 7, caractérisé en ce que les deux catalyseurs sont présents dans le mélange de catalyseurs dans un rapport tel que le rapport atomique $(Cu+Zn)/Co$ dans le mélange de catalyseurs soit compris entre 0,5 et 5.

3

9. Un procédé selon la revendication 7 ou 8, caractérisé en ce que la composition contenant du cuivre et du zinc a un rapport atomique Cu/Zn compris entre 0,25 et 4.

10. Un procédé selon l'une quelconque des revendications 1—9, caractérisé en ce qu'il est mis en oeuvre à une température de 125—350°C et une pression de 5—100 bars.

9